# EUROPEAN PATENT APPLICATION

(11) **EP 2 107 067 A1**
(43) Date of publication of application: **07.10.2009**
(21) Application number: 08154034.6
(22) Date of filing: 03.04.2008
(51) Int. Cl.: C07K 1/08, C07K 5/06

(54) **Boron catalysed peptide synthesis**

(71) Applicant: DSM IP Assets B.V., 6411 TE Heerlen (NL)
(72) Inventor: Quaedflieg, Peter Jan Leonard Mario, 6181 KA, ELSLOO (NL); Nuijens, Timo, 6160 MA, Geleen (NL); Vries , de, Johannes Gerardus (Hans), 6228 GZ, MAASTRICHT (NL); Kalkeren, van, Henri Antonius, 6669 AC, Dodewaard (NL)
(74) Representative: Hoogendam, Gerrie Christine

(57) **Abstract**

The invention relates to a method of synthesising a dipeptide, an oligopeptide or polypeptide comprising coupling a first amino acid or peptide to a second amino acid or peptide in the presence of a boron compound comprising a boron oxygen bond, which boron compound catalyses the formation of a peptidic bond, wherein one of the stereoisomers is formed in excess of the other stereoisomer or stereoisomers.

## Description

The invention relates to a method for synthesising a dipeptide, an oligopeptide or a polypeptide.

Peptides have many applications, for instance as a pharmaceutical, a food or feed ingredient, an agrochemical or a cosmetic ingredient.

For the purpose of this invention, with peptides is meant any chain of two or more α-amino acids. Hereinafter, when referred to an amino acid, an α-amino acid is meant, unless specified otherwise. For the purpose of this invention, the term 'peptides' includes dipeptides, oligopeptides and polypeptides. A dipeptide is based on two amino acids. The term 'oligopeptide' is used for peptides based on 3-200 amino acids, in particular based on 3-100, more in particular based on 3-50 amino acids, in particular any linear or cyclic chain of 3-200 amino acids, more preferably of 3-100 or 3-50 amino acids. The term 'polypeptides' is used for peptides based on a higher number of amino acids than specified for oligopeptides. When referred herein to an amino acid or a peptide, amino acids respectively peptides of which one or more amino functions, one or more carboxylic acid functions, and/or one or more other functions, such as one or more side group functions (*e*.*g*. hydroxyl functions, thiol functions and the like) which may be present, are protected are meant to be included, unless indicated otherwise.

Peptides may be synthesised by known chemical peptide synthesis as described for instance by N. Sewald and H.-D. Jakubke in "Peptides: Chemistry and Biology" as published by Wiley-VCH in 2002 (ISBN 3-527-30405-3), sections 4.1, 4.2, 4.3 and 4.4 (pages 135 - 208). In these chemical methods, an N-terminal amine protected amino acid or peptide having a free C-terminal carboxylic moiety is coupled with a C-terminal carboxyl protected amino acid or peptide having a free N-terminal amine moiety. In known chemical peptide synthesis always an at least (almost) stoichiometric amount of a coupling reagent is required, usually combined with activators or bases as for instance explained by N. Sewald and H.-D. Jakubke in "Peptides: Chemistry and Biology" as published by Wiley-VCH in 2002 (ISBN 3-527-30405-3), section 4.3 (pages 184 - 204). For instance, isobutyl chloroformate and pivaloyl chloride may be used in combination with a tertiary amine or carbodiimides like N,N-dicyclohexyl carbodiimide (DCC) and 1-ethyl-3-(3-dimethylaminopropyl)-carbodiimide hydrochloride (EDC) combined with 1-hydroxy-1H-benzotriazole (HOBt) or 1-hydroxy-7-aza-1H-benzotriazole (HOAt). Other examples of frequently used coupling reagents are phosphonium reagents or uronium reagents. Usually, the coupling reagents are expensive and/or toxic and give rise to much waste since they are needed in at least (almost) stoichiometric amounts. Additionally, since the reagents are reactive chemicals, usually the reactive functionalities on the amino acid side chain groups should be protected, such as the side chain functionalities of aspartic acid, glutamic acid, lysine, histidine, arginine, serine, threonine, asparagine, glutamine, tyrosine, tryptophan and cysteine. Protection and deprotection of the side chain functionalities is laborious, time consuming and cost-inefficient. Nevertheless, even if all side chain functionalities are protected, several side reactions may occur during the formation of the peptidic bond. After each coupling step a laborious aqueous (usually acidic and/or alkaline) work-up is required to get rid of the waste of coupling reagents and auxiliaries and of the side-products. In some cases laborious purification techniques such as chromatography are required to purify the desired (protected) peptide. During the aqueous work-up and other required purification steps, a considerable amount of desired peptide product may be lost, rendering the process cost-inefficient.

Further, in general known chemical methods to synthesise peptides require active cooling (usually to 10 °C or less, *e*.*g*. down to -30 °C) in order to sufficiently suppress side-reactions, such as racemisation or epimerisation.

Thus, there remains a need for alternative methods for peptide synthesis. Such methods may, *e*.*g*., increase flexibility in peptide synthesis and/or allow the synthesis of specific peptides that are not or relatively difficult to obtain using known methodology.

It is an object of the invention to provide a method that may serve as an alternative to known chemical methods, in particular to provide a method that overcomes one or more of the above described disadvantages. One or more other objects that may be met will be apparent from the description below.

It has now been found possible to synthesise a dipeptide, an oligopeptide or a polypeptide using a specific type of catalysts, capable of catalysing peptidic bond formation.

Accordingly, the present invention relates to a method of synthesising a dipeptide, an oligopeptide or a polypeptide comprising coupling a first amino acid or peptide to a second amino acid or peptide in the presence of a boron compound, which boron compound comprises a boron oxygen bond, said boron compound catalysing the formation of a peptidic bond, wherein one of the stereoisomers is formed in excess of the other stereoisomer or - in case more than two stereoisomers of the peptide exist - in excess of each of the other stereoisomers (alone or taken together).

In addition, the invention relates to a method for synthesising a cyclic oligopeptide or a cyclic polypeptide, comprising forming a peptidic bond between the N-terminal amino group and the C-terminal carboxylic acid group of an oligopeptide or polypeptide, in the presence of a boron compound, said boron compound comprising a boron oxygen bond, which boron compound catalyses the formation of a peptidic bond, wherein one of the stereoisomers is formed in excess of the other stereoisomer or- in case more than two stereoisomers of the peptide exist - in excess of each of the other stereoisomers (alone or taken together).

In addition, the invention relates to the use of a boron compound comprising a boron oxygen bond as a catalyst in the preparation of a stereomerically pure peptide.

The first amino acid or peptide and the second amino acid or peptide may be referred herein after as "starting compound" or "starting compounds".

Compared to known chemical methods, such as described above, a method of the invention may in particular provide a cheaper, more efficient method. Further, no stoichiometric amounts of reagents to couple the reactants (the peptide(s)/amino acid(s) to be coupled) are required; thereby the production of waste is reduced. It is also envisaged that the isolation of the synthesised peptide is simpler. Further, it is envisaged that the coupling can be carried out adequately, without having to protect all the side chain functionalities of the amino acids mentioned above when discussing known chemical methods.

Also, a method according to the invention can suitably be carried out under essentially water-free conditions, in particular in a solvent comprising less than 2 wt. % water, more in particular less than 1 wt. % water, less than 0.5 wt. % water, or less than 0.1 wt. % water.

In addition, a method according to the invention can suitably be carried out without needing active cooling.

The boron compound generally has a high chemical stability, under suitable coupling conditions, which allows the catalyst to be re-used a plurality of times, whilst catalytic activity is substantially maintained. In particular, the boron compound can withstand extreme conditions, such as high temperature (*e*.*g*. above 100 °C), without being substantially inactivated. This is advantageous with respect to flexibility in choosing reaction conditions. It also facilitates choosing separation conditions for separating the synthesised peptide and the boron compound and/or for separating the boron compound from the reaction medium, before, during or after recovery of the synthesised peptide.

The boron compound may for instance be recovered from the reaction mixture by liquid-liquid extraction, by forming a polar liquid phase and an apolar (or less polar) liquid phase, wherein the boron compound has a high affinity for the polar phase and the synthesised peptide has affinity for the apolar (or less polar) phase. The polar phase may for instance comprise one or more polar liquids selected from the group of water, dimethylformamide (DMF), dimethylsulfoxide (DMSO), N-methyl pyrrolidinone (NMP), and ionic liquids. The apolar phase is typically chosen such that it at least substantially does not dissolve in the polar phase. The apolar phase may for instance comprise one or more apolar liquids selected from the group of aromatic and aliphatic hydrocarbons. Such hydrocarbons may optionally comprise one or more substituents (as long as the substituent does not render the hydrocarbon substantially soluble in the polar phase). For instance, the substituent may be a halogen atom. Examples of (optionally substituted) hydrocarbons that may be used are toluene, xylene, n-pentane, n-hexane, n-heptane, cyclopentane and fluorobenzene.

After the liquid-liquid extraction, the peptide respectively boron compound can be recovered from the apolar phase (or less polar phase) respectively from the polar phase, in a manner known in the art, *e*.*g*., by evaporation of the liquid phase.

Further, a method according to the invention is versatile in that various amino acids or peptides can be coupled to each other. In principle, a method of the invention can be used to couple any two amino acids, to couple any amino acid to any terminal amino acid residue of a peptide, or to couple any N-terminal amino acid residue of a peptide to any C-terminal amino acid residue of a peptide.

Moreover, it has surprisingly been found possible to carry out a method according to the invention, starting from starting compounds that have a high degree of enantiomeric and/or diastereomeric purity, without complete racemisation or epimerisation. It is noted that amidation catalysts for catalysing the formation of amides by reacting a carboxylic acid and an amine are known in the art. In Synthesis 2001, No 11, 28 August 2001, pages 1611-1613, Latta *et al*. describe the use of a pyridineboronic acid derivative attached to a polystyrene solid support for coupling a carboxylic acid and an amine. Further, reference is made to preliminary studies relating to the synthesis the dipeptide Z-Phe-Ala-O^{t}Bu without specifying reaction conditions. Latta *et al*. state that complete racemisation of the Phe-residue had occurred, *i*.*e*. no stereoisomer had been formed in excess over the other three stereoisomers.

In a method according to the invention, in particular starting compounds may be used wherein one of the enantiomers of the amino acid or one of the diastereomers of the peptide of which the N-terminal amino group participates in the coupling and one of the enantiomers of the amino acid or one of the diastereomers of the peptide of which the C-terminal carboxyl group participates in the coupling are present in an enantiomeric excess (e.e.) or a diastereomeric excess (d.e.) of 90-100 %, preferably of 95-100 %, more preferably of 99-100 %, in particular of 99.5-100 %, more in particular of 99.8-100 %, to provide one of the enantiomers or diastereoisomers of the formed peptide in an enantiomeric excess or a diastereoisomeric excess. Compounds having an e.e. or a d.e. of 99.0-100 %, in particular 99.5-100 %, more in particular of 99.8-100 % may hereinafter also referred to as "stereomerically pure".

Usually, in a method of the invention a desired stereoisomer is formed with an e.e. or a d.e. of more than 25 %. In particular, the e.e. or the d.e. may be at least 30 %, at least 50 %, at least 65 %, at least 85 %, at least 90 % or at least 95 %. It is envisaged that at least in some embodiments, preparing a peptide in an e.e. or a d.e. of 98 % or more, 99 % or more, or 99.5 % or more, is feasible, if starting from compounds that are stereomerically pure.

For the purpose of this invention, with "stereoisomers" are meant compounds which have the same molecular formula and the same sequence of covalently bonded atoms, but different spatial orientations of those atoms.

For the purpose of this invention, with "chiral centre" is meant a grouping of atoms consisting of a central atom and distinguishable covalently bound atoms, such that the interchange of any two of the covalently bound atoms leads to a stereoisomer. Typically, a chiral centre is a carbon atom bearing four different covalently bound atoms.

For the purpose of this invention, with "enantiomers" are meant stereoisomers that are nonsuperimposable complete mirror images of each other.

For the purpose of this invention, with "diastereoisomers" (or "diastereomers") are meant stereoisomers which are not enantiomers.

Within the context of the present invention the term "organic moiety" is in particular meant to include linear or branched, optionally substituted alkyl, alkenyl or alkynyl groups; optionally substituted cycloalkyl groups which optionally have one or more unsaturated, exocyclic or endocyclic, carbon carbon bonds; optionally substituted aryl groups; and optionally substituted aralkyl groups. Alkyl groups may in particular comprise 1-12 carbons. Alkenyl or alkynyl groups may in particular comprise 2-12 carbons. Cycloalkyl groups or aryl groups may in particular comprise 3-18 carbons more in particular 3-12 carbons. Aralkyl groups may in particular comprise 5-19 carbons, more in particular 5-13 carbons.

An alkyl, alkenyl or alkynyl group optionally comprises one or more heteroatoms in a chain thereof. A cycloalkyl, aryl or aralkyl group optionally comprises one or more heteroatoms in a ring thereof. The heteroatom may in particular be selected from the group of B, S, N, O, P, Si, Se, more in particular from B, S, O and N.

If substituted, an organic moiety may in particular be substituted with one or more functional groups comprising one or more heteroatoms, which heteroatoms may in particular be selected from the group of halogens, P, Si, B, O, S, Se and N. Substituents include in particular halogen atoms, partially halogenated alkyl groups, fully halogenated alkyl groups, partially halogenated alkoxy groups, fully halogenated alkoxy groups, amino groups, carbonyl groups, imine groups, carboxylic acid groups and sulphonic acid groups.

More specifically, a substituent may be selected amongst the group of F, Cl, Br, I, optionally substituted C₁₋₆ alkyl, optionally substituted C₂₋₆ alkenyl, optionally substituted C₂₋₆ alkynyl, optionally substituted C₁₋₆ alkoxy, optionally substituted C₁₋₆ alkylthio, optionally substituted C₃₋₆ cycloalkyl, optionally substituted OC₃₋₆ cycloalkoxy, optionally substituted OCH₂-phenyl, OH, NO₂, CN, phenyl, and NR^{x}R^{y}. Herein, R^{x} and R^{y} are organic moieties which may in particular independently be selected from the group of H, optionally substituted C₁₋₄ alkyl, optionally substituted cycloalkyl, phenyl, benzyl and phenetyl.

When referred herein to an acid both the neutral and its ionised form or forms (its conjugated base(s)) are meant to be included. Also salts of an acid and anhydrides of the acid are meant to be included in the term "acid".

The boron compound may in particular be a boron based acid or an organoborane.

As used herein a boron based acid is in particular a compound represented by the following structural formula.

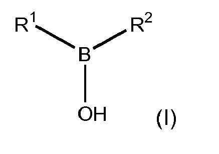

Herein R¹ and R² are independently selected from the group of hydroxyl and organic moieties. R¹ and R² may both be a hydroxyl, in which case the compound is boric acid. In case one of R¹ and R² is a hydroxyl and the other an organic moiety, the compound may be referred to as a boronic acid. In case R¹ and R² both are organic moieties, or if the R¹ and R² together form a single organic moiety, such as an organic ring structure, the compound may be referred to as a borinic acid.

In particular R¹ and/or R² may independently be selected from the group of hydroxyl, optionally substituted alkyl, optionally substituted aryl, optionally substituted cycloalkyl, optionally substituted alkoxy and optionally substituted aryloxy.

As used herein an organoborane in particular is a compound represented by the following structural formula.

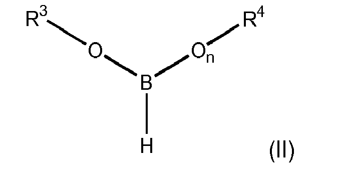

Herein R³ and R⁴ are independently selected from the group of hydrogen and organic moieties. In an embodiment, R³ and R⁴ together form an organic ring structure. The integer n is 0 or 1, Preferably, n is 1.

In particular R³ and/or R⁴ may independently be selected from the group of optionally substituted alkyl, optionally substituted aryl and optionally substituted cycloalkyl.

In a preferred embodiment the boron compound is an aromatic compound, which optionally contains one or more further heteroatoms. The aromatic compound typically comprises one or more aromatic rings. One or more of said rings may be free of any heteroatoms. In an embodiment, one or more of said rings comprise one or more electron withdrawing moieties in the ring. Preferably one or more of the rings of the aromatic compound comprise one or more electron withdrawing groups as substituents attached to the ring, in addition to the boron moiety. Thus, in a preferred embodiment, one or more rings comprise 2, 3, 4, 5 or 6 electron withdrawing groups as substituents, on the ring.

Preferred electron withdrawing substituents in a boron compound used in a method of the invention, are substituents selected from the group of F, Cl, Br, I, halogenated alkyl groups and halogenated alkoxygroups. The halogenated alkyl or alkoxy group may be represented by the formula O_{w}-CₓH_{y}X_{z}, wherein each X independently represents F, Cl, Br or I; w is 0 or 1; x is an integer of at least 1, preferably chosen in the range of 1-12, in particular in the range of 1-6; and y+z equals 2x+1. Preferably the number of halogen atoms is larger than the number of hydrogen atoms, more preferably z equals 2x+1 (*i*.*e*. y=0). In a specific embodiment, the halogenated alkyl is CF₃, C₂F₅ or C₃F₇. An alkoxy group may in particular be present at the meta position, relative to the boron moiety.

In a particularly preferred embodiment, an aryl boronic acid compound is used. The term "aryl boronic acid" is used herein to refer to a compound comprising a boronic acid group, which is directly bound to an aromatic ring of an aromatic moiety (see Formula I, wherein R¹ is OH and R² is an aryl.) The aryl group may in particular comprise 6-18 carbon atoms, more in particular 6-12 carbon atoms.

The term "aromatic" as used in this application is meant to refer to a conjugated ring system of unsaturated bonds, lone pairs, or empty orbitals, which exhibit a stabilisation stronger than would be expected by the stabilisation of conjugation (alternate double and single bonds) alone. The aromatic has a delocalised conjugated π-system, a coplanar structure, contributing atoms arranged in one or more rings, and 4*n*+2 delocalised π-electrons (wherein *n* is a non-negative integer). Examples of aromatics include acridine, anthracene, benzene, benzimidazole, benzofuran, benzopyrazole, benzopyridine, benzothiophene, benzoxazole, carbazole, dibenzofuran, dibenzothiophene, furan, imidazole, isoxadiazole, isoxazole, naphthalene, oxadiazole, oxazole, phenanthrene, phenanthridine, phenanthroline, phenazine, phenothiazine, purine, pyrazine, pyrazole, pyridazine, pyridine, pyrimidine, pyrole, quinazoline, quinoline, quinoxaline, thiadiazole, thiophene, and xanthene.

In particular the aryl boronic acid may be selected from the group of dihalogen phenylboronic acids, trihalogen phenylboronic acids, tetrahalogen phenylboronic acids and pentahalogen phenylboronic acids. More in particular the aryl boronic acid may be selected from 2,6-difluorophenylboronic acid, 3,4,5-trifluorophenylboronic acid, pentafluorophenyl boronic acid, 4-bromo-2,3,5,6 tetrafluorophenylboronic acid, 3,5-bis(trifluoromethyl)phenyl boronic acid, 3, 5-bis(perfluorodecyl)phenyl boronic acid, dimesitylene borinic acid and 4,5,6,7-tetrachlorobenzo[d][1,3,2]dioxaborole. In particular, good results have been achieved with 2,6-difluorophenylboronic acid or with 3,4,5-trifluorophenylboronic acid, 2-bromophenyl boronic acid, 4,5,6,7-tetrachlorobenzo[d][1,3,2]dioxaborole and dimesitylene borinic acid.

The aryl boronic acid can for instance comprise an optionally substituted ring system, which rings system may be monocyclic or polycyclic, with at least 2, at least 3, at least 4, at least 5 or at least 6 carbon atoms. One or more heteroatoms, may be present in the ring, in particular one or more heteroatoms selected from N, S and O, provided that the aromatic character is maintained. The upper limit for the number of atoms forming the ring system is in principle not critical, as long as the ring system does not hinder the reaction to an unacceptable extent. In particular the number of atoms forming the ring system may be 20 or less, 14 or less, or 12 or less, *e*.*g*. 6.

If an organoborane is used, R³ and R⁴ preferably form an aryl together, in particular a phenyl. Preferably, the aryl, in particular the phenyl, comprises one or more further electron withdrawing substituents, in particular one or more electron withdrawing substituents as mentioned above. More in particular the substituents may be selected from F, Cl, Br, I, halogenated alkyl groups and halogenated alkoxygroups, such as those described above. In a specific embodiment, the organoborane is selected from perchlorocatecholborane and perfluorocatecholborane.

Dependent on the catalytic activity and a desired coupling rate the catalyst concentration may be added in a relatively low or relatively high concentration. Usually, the concentration is at least 0.01 mol %, in particular at least 0.1 mol %, more in particular at least 0.5 mol %, or at least 1.0 mol %, based on the molar concentration of the first amino acid or peptide and the second amino acid or peptide, taken together.

The upper limit is in principle not critical. In view of cost-efficiency and recovery efficiency (in relation to separation of the product and the catalyst, which may be re-used), the concentration is usually 100 mol % or less, in particular 50 mol % or less, more in particular 10 mol % or less, based on the molar concentration of the first amino acid or peptide and the second amino acid or peptide, taken together.

An amino acid used for synthesising the dipeptide, the oligopeptide or the polypeptide may be proteinogenic or non-proteinogenic. A peptide used for synthesising the oligopeptide or the polypeptide may be formed of proteinogenic amino acids, non-proteinogenic acids or a combination of one or more proteinogenic amino acids and one or more non-proteinogenic acids. Proteinogenic amino acids are the amino acids that are encoded by the genetic code. Proteinogenic amino acids include: alanine, valine, leucine, isoleucine, serine, threonine, methionine, cysteine, asparagine, glutamine, tyrosine, tryptophan, glycine, aspartic acid, glutamic acid, histidine, lysine, arginine, proline and phenylalanine. Examples of non-proteinogenic amino acids are phenylglycine and 4-fluoro-phenylalanine.

Although the invention may be used for coupling two identical amino acids or peptides, the invention is also particularly suitable to couple amino acids or peptides that are different from each other.

In an embodiment the first amino acid or peptide is an N-terminal protected amino acid or peptide and/or the second amino acid or peptide is a C-terminal protected amino acid or peptide.

In addition, at least in some embodiments, the method can be carried out without needing to protect amino acid side functionalities in order to avoid the risk of excessive side-reactions. This is in particular an advantage over known chemical peptide synthesis. In particular, at least in some embodiments side groups of one or more amino acid (residues) selected from histidine, arginine, serine, threonine, tryptophan, tyrosine, asparagine, glutamine, methionine and cysteine do not need to be protected in order to avoid the risk of excessive reaction between the side group and another functional group in the reaction medium, although such groups may be protected if desired. In order to reduce the risk of undesired side reactions, in particular side groups of aspartic acid, glutamic acid and/or lysine, are preferably protected. Suitable protecting groups for the side-chain functionalities, for the N-terminal amino groups and for the C-terminal carboxylic acid groups are generally known in the art, *e*.*g*. from N. Sewald and H.-D. Jakubke in "Peptides: Chemistry and Biology" as published by Wiley-VCH in 2002 (ISBN 3-527-30405-3), Chapter 4.

Carboxyl groups can for instance be protected as their ester, in particular as their alkyl ester, such as a C₁-C₅ ester or their benzyl ester. Alternatively, the carboxyl group can be amidated to form a carboxyamide. In particular, good results have been achieved with a method wherein the C-terminal carboxylic acid is esterified to form a methyl ester or a *t*-butyl ester.

Examples of suitable N-protecting groups include carbonyl type protecting groups, for instance 'Z' (*i*.*e*. benzyloxycarbonyl), 'Boc' (*i*.*e*. *t*-butyloxycarbonyl), 'For' (*i*.*e*. formyl) and 'PhAc' (*i*.*e*. phenacetyl).

In a specific embodiment, at least one of the starting compounds is a peptide synthesised by a method of the invention. For instance, to a peptide synthesised in a method of the invention, a further amino acid can be coupled. The invention also allows coupling of two or more oligopeptides, which have been prepared in the presence of the boron compound, to each other in the presence of a boron compound - catalysing the coupling. In particular in such an embodiment, the oligopeptides may be selected from oligopeptides formed of 2-10 amino acids, more in particular from the group of dipeptides, tripeptides and tetrapeptides. It is also possible to couple a peptide synthesised in a method according to the invention to a further peptide by a coupling technique other than by using a boron compound as defined herein, to form a larger peptide, which technique may be known in the art *per se*, *e*.*g*. chemical ligation, using stoichiometric coupling agents.

Suitable concentrations for the first amino acid or peptide and the second amino acid or peptide, which are to be coupled, may in principle be chosen within wide limits. Usually the molar ratio of nucleophilic amino acid or peptide (*i*.*e*. providing the N-terminal amino group for the coupling) to electrophilic amino acid or peptide (*i*.*e*. providing the C-terminal carboxylic acid group for the coupling) is at least 0.5, in particular at least 0.8, more in particular at least 0.9. In a preferred method said ratio is about 1.0 (to provide stoichiometric conditions) or larger than 1.0. In a particularly preferred method said ratio is 1.1 or more, more in particular 1.2 or more. An excess of nucleophilic amino acid or peptide compared to the electrophilic amino acid or peptide is in particular preferred for improving yield and/or obtaining the desired synthesised peptide in a high e.e. or d.e..

Usually the molar ratio of nucleophilic amino acid or peptide to electrophilic amino acid or peptide is 10 or less, in particular 5 or less, more in particular 2.5 or less, 1.5 or less, or 1.3 or less.

In terms of concentrations, the total concentration of the first amino acid or peptide and the second amino acid or peptide is usually at least 0.1 wt. %, preferably at least 1 wt. % in particular at least 10 wt. %, based on total weight of the reaction medium. In general, the concentration may be up to the saturation concentration at which these starting compounds dissolve, although in principle it is not necessary that the starting compounds fully dissolve. In particular said total concentration may be up to 30 wt. %, more in particular up to 25 wt. %.

Suitable reaction media are in particular organic liquids and ionic liquids, wherein the compounds to be coupled are at least partially soluble at the used concentration.

A suitable organic liquid may in particular be selected from the group of aprotic solvents, such as from the group of toluene, xylene, mesitilene, anisole, fluorobenzene, chlorobenzene, 1,4-dioxane, 4-methyl-2-pentanone, N,N-dimethylformamide, 1,2-dichloroethane, methylethylketone, cyclohexane, methyl vinyl ketone, NMP and the like, including mixtures of two or more of said liquids. Good results have in particular been achieved with fluorobenzene and with toluene.

Usually, water (formed as a side-product during the coupling) is removed during the coupling. This is desired for an efficient synthesis.

In an embodiment, water is removed by evaporation (*e*.*g*. distillative). For efficient water removal in this embodiment, the reaction medium may have a higher boiling point than water and/or form an azeotrope with water.

In particular in case water is to be removed by evaporation, a reaction medium may be chosen that has a higher boiling point than water, *i*.*e*. above 100 °C at 1 atm., *e*.*g*. of at least 110 °C. Examples of such media are toluene, xylene, mesitilene, anisole, chlorobenzene, N,N-dimethyl-formamide and NMP.

In a particularly preferred embodiment, the reaction medium forms an azeotrope with water, preferably at a water to reaction medium ratio (wt. to wt.) of 1:99 or more, in particular of 3:97 or more, more in particular of 5:95 or more. The lower limit of said ratio may be as low as 99:1 or even lower, in particular said ratio may be 25:75 or more, more in particular 50:50 or more. By carrying out the method in a reaction medium wherein the water content is below the water content in the azeotrope, water (formed in the coupling and/or water present as an impurity in the reaction medium) can easily be removed by evaporation, also if the boiling point of the reaction medium is below the boiling point of water. Reaction media that form an azeotrope with water are generally known in the art. Such media include toluene, fluorobenzene, cyclohexane, xylene, 1,2-dichloroethane, methyl ethyl ketone and methyl vinyl ketone.

In a specific embodiment, an ionic liquid is used. An ionic liquid is a liquid formed of a salt that is liquid under the process conditions, such as a melt of a salt. Ionic liquids generally have a boiling point well over 100 °C and a low vapour pressure below the boiling point, allowing easy and efficient removal of water by evaporation. The ionic liquid used in a method of the invention, has a melting point below the reaction temperature, usually a melting point below 100 °C, preferably of 50 °C or less, in particular of 25 °C or less. Such liquid may be referred to as a room temperature liquid salt. Salts that form an ionic liquid are known in the art.

For instance, US-A 4,764,440 discloses ionic liquids comprising a mixture of a metal halide and a hydrocarbyl-saturated onium salt, wherein at least one of the hydrocarbyl groups is an aromatic hydrocarbyl group.

US-A 5,892,124 discloses liquid salts of the general formula Q⁺A⁻, wherein Q⁺ represents quaternary ammonium or phosphonium, and A⁻ represents various anions including tetrachloroaluminate and trichlorozincate.

WO 02/26381 describes various ionic liquids, including ionic liquids comprising a salt of the following cations and/or anions:
- cations selected from the group of monosubstituted imidazolium compounds, disubstituted imidazolium compounds, trisubstitued imidazolium compounds, pyridinium compounds, pyrrolidinium compounds, phosphonium compounds, ammonium compounds, guanidinium compounds and isouronium compounds, including combinations thereof.
- anions selected from the group of chloride, bromide, iodide, nitrate, nitrite, fluoride, phosphate, imide, amide, borate, tosylate, tetrafluoroborate, hexafluoroborate, hexafluorophosphate, trifluoromethanesulfonate, methylsulphate, bis(pentafluoroethyl)phosphinate, thiocyanate, octylsulphate, hexylsulphate, butylsulphate, ethylsulphate, hexafluoroantimonate, bis-(pentafluoroethyl)phospinate, bis-(trifluoromethyl)imide, trifluoroacetate, bis-trifluorsulfonimide, triflate and dicyanamide, including combinations thereof.

The coupling can be carried out in a single solution phase, wherein the compounds to be coupled or the compound to be cyclised and the boron compound is dissolved in a solvent, in particular an organic liquid or ionic liquid, as mentioned above.

It is also possible to make use of a heterogeneous system, wherein the boron compound is immobilised onto a support, in particular a solid support. Suitable supports onto which a boron compound can be immobilised and manners for immobilising the boron compound may be based on those known in the art. For instance, the boron compound may be immobilised onto beads or onto a monolithic structure. Suitable carriers include polymers such as polystyrene and the like.

The temperature is chosen sufficiently high to allow the coupling to proceed at a desired rate. Usually, the coupling takes place while heating. Usually the temperature exceeds 30 °C. In particular the temperature may be 50 °C or more, or 80 °C or more. The temperature is typically chosen at or below the boiling point (at the existing pressure) of the medium wherein the coupling normally takes place. In order to avoid substantial decomposition of the peptide that is formed, and/or in view of an efficient energy consumption, and/or for synthesising a peptide of interest in a high e.e. or d.e., the temperature preferably is below 170 °C, more preferably 150 °C or less. In a particularly preferred method, the temperature is 120°C or less.

As indicated above, water may be removed by evaporation, such as by distillation and/or azeotropic water removal. In a further embodiment, water is removed using a material that adsorbs water and/or using a chemical reaction wherein water is one of the reactants. In an embodiment wherein a water adsorbing material or a material that reacts with water is used, the reaction medium may be a liquid as described above, or a different reaction medium. A suitable water-adsorbing material is a molecular sieve (molsieve) which adsorbs water or another hygroscopic material. Suitable molsieves are commercially available and include molsieves having a pore size in the range of 3-4 A (*e*.*g*. available from Acros). Other hygroscopic materials are generally known in the art. A suitable way of removing water is by a chemical reaction, *e*.*g*., by reacting the water with an (inorganic) hydride, such as calcium hydride.

In an embodiment, water is continuously removed. In particular, a Dean-Stark apparatus, a Soxhlet apparatus or the like may be used. Herein, the coupling in the presence of the boron compound is typically carried out under conditions, whereby reaction medium is refluxed while water is removed continuously. The pressure may be atmospheric, sub-atmospheric or super-atmospheric. By increasing respectively decreasing the pressure, the reflux temperature, and thereby the reaction temperature, can be increased respectively decreased. Usually the pressure is at least 1 mbara, preferably at least 5 mbara. Usually the pressure is 10 bara or less, preferably about 1 bara or less.

Two or more ways of water removal may be used, in combination. For example distillation may be used in combination with an adsorbent. In such embodiment reaction medium (usually an organic solvent) containing water is evaporated and the evaporated liquid is treated with the adsorbent, optionally after condensation, to remove the water. The dried reaction medium may thereafter be led back to the reaction mixture. For such an embodiment, a Soxhlet apparatus comprising an adsorbent (such as molecular sieves) or a compound that reacts with water (such as a hydride) is particularly suitable. Direct contact of adsorbent with the boron compound and the reactants is thus avoided, which is desired in case the adsorbent may interfere with the boron compound, the reactants or the formed product, which may be for instance the case if a hydride is used.

In an advantageous embodiment, water is continuously or intermittently removed by evaporation, in particular by distillation, *e*.*g*. vacuum distillation, whilst continuously or intermittently fresh reaction medium (having a lower water content than the reaction mixture wherein water is being formed as a result of the coupling) is added. The amount of added reaction medium is typically sufficient to replace reaction medium that is evaporated, together with the water that is evaporated. Such embodiment is in particular advantageous in view of a high reaction rate, whilst obtaining a peptide with a satisfactory d.e. or e.e.

The invention will now be illustrated by the following examples.

### EXAMPLES

### GENERAL

Chemicals were obtained from commercial sources and used without further purification. ¹H NMR spectra were recorded on a Bruker Avance 300MHz NMR (300.1 MHz) spectrometer and chemical shifts are given in ppm (δ) relative to CDCl₃ (7.26 ppm). 3A molecular sieves (molsieves) (8 to 12 mesh, Acros) were activated under reduced pressure at 200°C.

Analytical HPLC method 1 (for monitoring the conversions of the coupling reactions and determining the d.e. of Z-Phe-Leu-NH₂) was performed on an HP1090 Liquid Chromatograph, using a reversed-phase column (Inertsil ODS-3, C18, 5 µm particle size, 150 mm length, 4.6 mm internal diameter) at 40°C. UV detection was performed at 220 nm using a UV-VIS 204 Linear spectrometer. The gradient program was: 0-25 min linear gradient ramp from 5% to 98% eluent B and from 25-30 min at 5% eluent B (eluent A: 0.5 mL/L methane sulfonic acid (MSA) in H₂O, eluent B 0.5 mL/L MSA in acetonitrile). The flow was 1 mL/min from 0-25 min and 2 mL/min from 25-29.8 min, then back to 1 mL/min until stop at 30 min. Injection volumes were 20 µL. Rₜ (Z-Phe-Phe-O*^{t}*Bu) = 23.53 min, Rₜ (Z-Phe-OH) = 15.99 min, Rₜ (H-Phe-O*^{t}*Bu) = 8.53 min, Rₜ (Z-Phe-Leu-OMe) = 20.23 min, Rₜ (Z-Phe-Leu-O*^{t}*Bu) = 23.02 min, Rₜ (Z-D-Phe-L-Leu-NH₂) = 16.34 min, Rₜ (Z-L-Phe-L-Leu-NH₂) = 16.14 min, Rₜ (Phe) = 2.12 min.

Analytical HPLC method 2 (for determining the e.e. of Phe) was performed on a Crownether Cr(+) Daicel column (150 mm length, 4.0 mm internal diameter, 5µm particle size) at 25 °C with 10 mM aqueous HClO₄ (pH = 2.0) as the eluent. UV detection was performed at 210 nm using a UV-VIS 204 Linear spectrometer. The flow was 1 mL/min. Injection volumes were 5 µL. Rₜ (D-Phe) = 6.90 min, Rₜ (L-Phe) = 8.82 min.

Analytical HPLC method 3 (for determining the d.e. of Z-Phe-Leu-O*^{t}*Bu) was performed on 2 Lichrosphere diol columns (150 mm length, 4.6 mm internal diameter, 5 µm particle size) in series at 8 °C with *n*-heptane/*t*-butanol (98.75/1.25, v/v) as the eluent. UV detection was performed at 220 nm using a UV-VIS 204 Linear spectrometer. The flow was 1.6 mL/min. Injection volumes were 10 µL. Rₜ (Z-L-Phe-L-Leu-O*^{t}*Bu) = 16.30 min, Rₜ (Z-D-Phe-L-Leu-O*^{t}*Bu) = 17.61 min.

Analytical HPLC method 4 (for determining the d.e. of Z-Phe-Leu-OMe) was performed on 2 Lichrosphere diol columns (150 mm length, 4.6 mm internal diameter, 5 µm particle size) connected in series at 8 °C with *n*-heptane/*t*-butanol (93.25/6.75, v/v) as the eluent. UV detection was performed at 220 nm using a UV-VIS 204 Linear spectrometer. The flow was 1.6 mL/min. Injection volumes were 10 µL. Rₜ (Z-L-Phe-L-Leu-OMe) = 7.96 min, Rₜ (Z-D-Phe-L-Leu-OMe) = 10.04 min.

H-Phe-O*^{t}*Bu, H-Leu-OMe, H-Leu-O*^{t}*Bu and H-Leu-NH₂ were prepared from the corresponding commercial HCl salts (Bachem, Zwitserland) by dissolution into a mixture of sat. aqueous NaHCO₃ and EtOAc. After stirring vigorously for 1 hour, the layers were separated and the organic phase was concentrated *in vacuo* and the volatiles co-evaporated *in vacuo* with toluene. The resulting free amine was used as such for the reaction.

### EXAMPLE 1: SYNTHESIS OF Z-PHE-PHE-O^{T}BU USING 2,6-DIFLUOROPHENYLBORONIC ACID

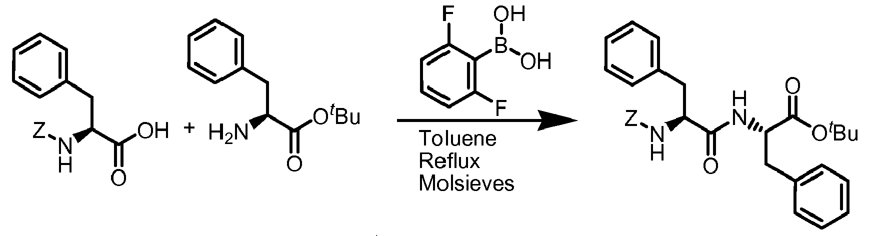

To 214 mg H-Phe-O*^{t}*Bu (L-phenylalanine *tert*-butylester, 0.97 mmol) were added 299 mg Z-Phe-OH (*N*-benzyloxycarbonyl-L-phenylalanine,1 mmol), 8 mg 2,6-difluorophenylboronic acid (0.05 mmol, 5 mol%) and 50 mL toluene. This mixture was heated under reflux for 16 h using a Dean-Stark distilling trap filled with 10 g of 3 A molsieves. HPLC (method 1) indicated that all H-Phe-O*^{t}*Bu had been converted and only Z-Phe-Phe-O*^{t}*Bu had been formed. After cooling to ambient temperature the mixture was diluted with 200 mL EtOAc and washed with sat. aqueous NaHCO₃ (2 x 200 mL), aqueous HCl (pH 2, 2 x 200 mL) and brine (100 mL), dried (Na₂SO₄) and concentrated *in vacuo* giving the pure dipeptide.

¹H-NMR (CDCl₃) δ 7.20-6.90 (m, 15 H), 6.43 (br s, 1H), 5.40 (br s, 1 H), 4.95 (s, 2H), 4.60 (m, 1H), 4.38 (m, 1H), 2.93 (m, 4H), 1.25 (s, 9H).

To assess the stereochemical purity, the product was dissolved in 100 mL 6 N aqueous HCl and heated under reflux for 4 h. HPLC (method 1) indicated that the product had been completely hydrolysed to Phe. After evaporation of the solvents *in vacuo* the residue was dissolved in a mixture of 50 mL H₂O/acetonitrile (1/1, v/v) and 5 mL MeOH. The enantiomeric excess of Phe was determined by HPLC (method 2) to be 75%. Assuming that only the N-terminal Phe residue had racemized this would mean that the e.e. of the N-terminal Phe residue is 50%.

### EXAMPLE 2: SYNTHESIS OF Z-PHE-LEU-O^{T}BU USING 2,6-DIFLUOROPHENYLBORONIC ACID

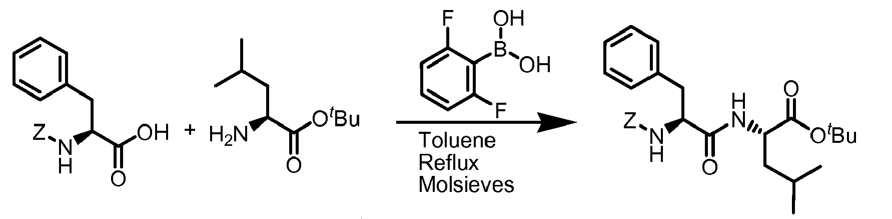

To 94 mg H-Leu-O*^{t}*Bu (L-leucine *tert*-butylester, 0.5 mmol) were added 150 mg Z-Phe-OH (0.5 mmol, 1 equiv.), 4 mg 2,6-difluorophenylboronic acid (0.025 mmol, 5 mol%) and 10 mL toluene. This mixture was heated under reflux for 16 h using a Dean-Stark distilling trap filled with 10 g of 3 A molsieves. HPLC (method 1) indicated that 56% Z-Phe-OH had been converted and only Z-Phe-Leu-O*^{t}*Bu had been formed. Analytical HPLC (method 3) indicated a d.e. of 43%.

### EXAMPLE 3: SYNTHESIS OF Z-PHE-LEU-O^{T}BU USING 2,6-DIFLUOROPHENYLBORONIC ACID

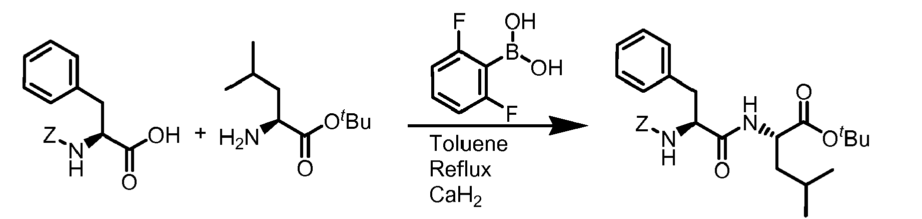

To 94 mg H-Leu-O*^{t}*Bu (0.5 mmol) were added 150 mg Z-Phe-OH (0.5 mmol, 1 equiv.), 4 mg 2,6-difluorophenylboronic acid (0.025 mmol, 5 mol%) and 10 mL toluene. This mixture was heated under reflux for 16 h using a dropping funnel with pressure equalizer filled with cotton wool and 3 g of CaH₂. HPLC (method 1) indicated that 57% Z-Phe-OH had been converted and only Z-Phe-Leu-O*^{t}*Bu had been formed. Analytical HPLC (method 3) indicated a d.e. of 36%.

### EXAMPLE 4: SYNTHESIS OF Z-PHE-LEU-O^{T}BU USING 3,4,5-TRIFLUOROPHENYLBORONIC ACID

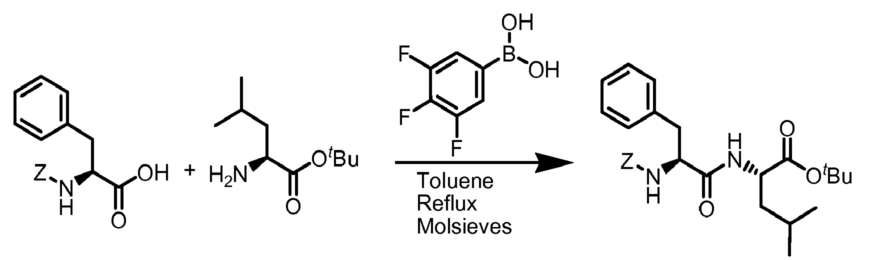

To 94 mg H-Leu-O*^{t}*Bu (0.5 mmol) were added 150 mg Z-Phe-OH (0.5 mmol, 1 equiv.), 4 mg 3,4,5-trifluorophenylboronic acid (0.025 mmol, 5 mol%) and 10 mL toluene. This mixture was heated under reflux for 16 h using a Dean-Stark distilling trap filled with 10 g of 3 A molsieves. HPLC (method 1) indicated that 54% Z-Phe-OH had been converted and only Z-Phe-Leu-O*^{t}*Bu had been formed. Analytical HPLC (method 3) indicated a d.e. of 55%.

### EXAMPLE 5: SYNTHESIS OF Z-PHE-LEU-O^{T}BU USING 3,4,5-TRIFLUOROPHENYLBORONIC ACID

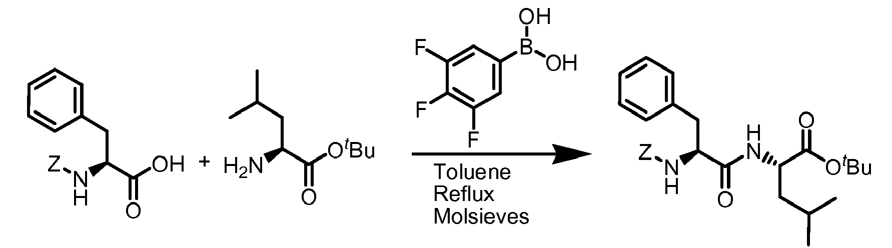

To 84 mg H-Leu-O*^{t}*Bu (0.45 mmol) were added 150 mg Z-Phe-OH (0.5 mmol, 1.1 equiv.), 4 mg 3,4,5-trifluorophenylboronic acid (0.025 mmol, 5 mol%) and 10 mL toluene. This mixture was heated under reflux for 16 h using a Dean-Stark distilling trap filled with 10 g of 3 A molsieves. HPLC (method 1) indicated that 55% Z-Phe-OH had been converted and only Z-Phe-Leu-O*^{t}*Bu had been formed. Analytical HPLC (method 3) indicated a d.e. of 55%.

### EXAMPLE 6: SYNTHESIS OF Z-PHE-LEU-O^{T}BU USING 3,4,5-TRIFLUOROPHENYLBORONIC ACID

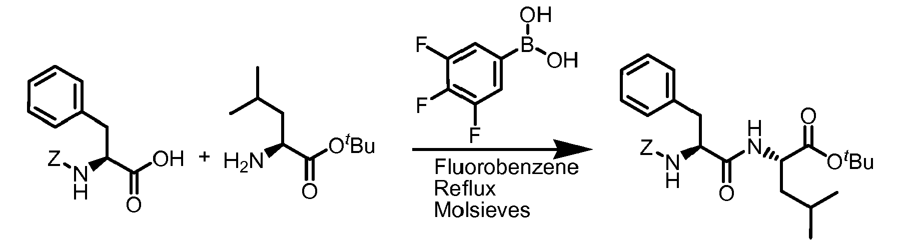

To 94 mg H-Leu-O*^{t}*Bu (0.5 mmol) were added 150 mg Z-Phe-OH (0.5 mmol, 1 equiv.), 4 mg 3,4,5-trifluorophenylboronic acid (0.025 mmol, 5 mol%) and 10 mL fluorobenzene. This mixture was heated under reflux for 16 h using a Dean-Stark distilling trap filled with 10 g of 3 A molsieves. HPLC (method 1) indicated that 34% Z-Phe-OH had been converted and only Z-Phe-Leu-O*^{t}*Bu had been formed. Analytical HPLC (method 3) indicated a d.e. of 84%.

### EXAMPLE 7: SYNTHESIS OF Z-PHE-LEU-OME USING 3,4,5-TRIFLUOROPHENYLBORONIC ACID

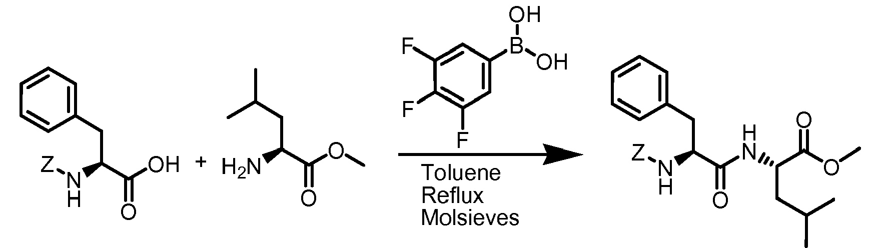

To 73 mg H-Leu-OMe (L-leucine methylester, 0.5 mmol) were added 150 mg Z-Phe-OH (0.5 mmol, 1 equiv.), 4 mg 3,4,5-trifluorophenylboronic acid (0.025 mmol, 5 mol%) and 10 mL toluene. This mixture was heated under reflux for 16 h using a Dean-Stark distilling trap filled with 10 g of 3 A molsieves. HPLC (method 1) indicated that 47% Z-Phe-OH had been converted and only Z-Phe-Leu-OMe had been formed. Analytical HPLC (method 4) indicated a d.e. of 75%.

### EXAMPLE 8: SYNTHESIS OF Z-PHE-LEU-OME USING 3,4,5-TRIFLUOROPHENYLBORONIC ACID

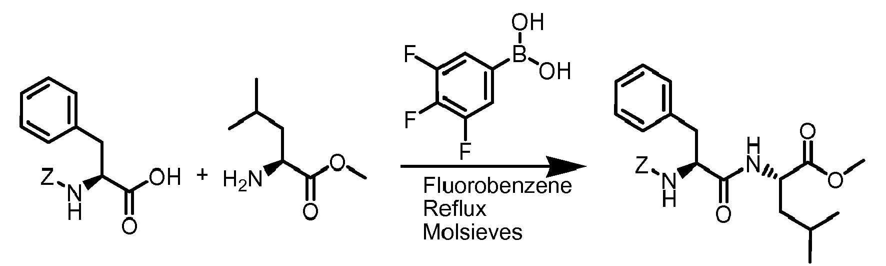

To 73 mg H-Leu-OMe (0.5 mmol) were added 150 mg Z-Phe-OH (0.5 mmol, 1 equiv.), 4 mg 3,4,5-trifluorophenylboronic acid (0.025 mmol, 5 mol%) and 10 mL fluorobenzene. This mixture was heated under reflux for 16 h using a Dean-Stark distilling trap filled with 10 g of 3 A molsieves. HPLC (method 1) indicated that 18% Z-Phe-OH had been converted and only Z-Phe-Leu-OMe had been formed. Analytical HPLC (method 4) indicated a d.e. of 86%.

### EXAMPLE 9: SYNTHESIS OF Z-PHE-LEU-O^{T}BU USING DIMESITYLENEBORINIC ACID

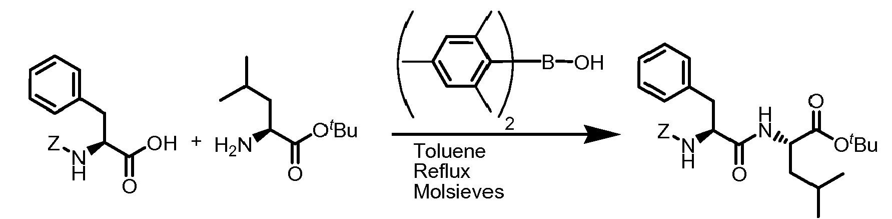

To 94 mg H-Leu-O*^{t}*Bu (0.5 mmol) were added 150 mg Z-Phe-OH (0.5 mmol, 1 equiv.), 7 mg dimesityleneborinic acid (0.025 mmol, 5 mol%) and 10 mL toluene. This mixture was heated under reflux for 16 h using a Dean-Stark distilling trap filled with 10 g of 3 A molsieves. HPLC (method 1) indicated that 64% Z-Phe-OH had been converted and only Z-Phe-Leu-O*^{t}*Bu had been formed. Analytical HPLC (method 3) indicated a d.e. of 53%.

### EXAMPLE 10: SYNTHESIS OF Z-PHE-LEU-NH₂ USING 2,6-DIFLUOROPHENYLBORONIC ACID

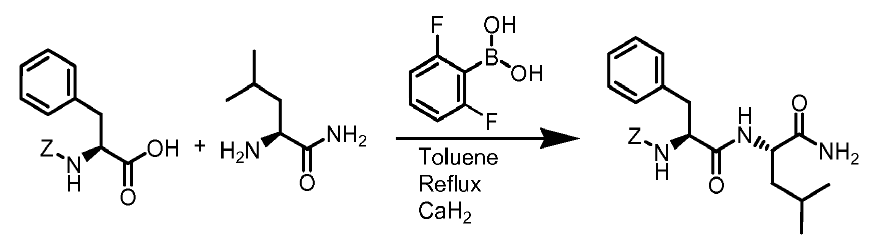

To 130 mg H-Leu-NH₂ (L-leucine amide, 1 mmol) were added 299 mg Z-Phe-OH (1 mmol, 1 equiv.), 8 mg 2,6-difluorophenylboronic acid (0.05 mmol, 5 mol%) and 20 mL toluene. This mixture was heated under reflux for 16 h using a dropping funnel with pressure equalizer filled with cotton wool and 3 g of CaH₂. HPLC (method 1) indicated that 50% Z-Phe-OH had been converted and only Z-Phe-Leu-NH₂ had been formed. Analytical HPLC (method 1) indicated a d.e. of 35%.

### EXAMPLE 11: SYNTHESIS OF Z-PHE-LEU-NH₂ USING BORIC ACID

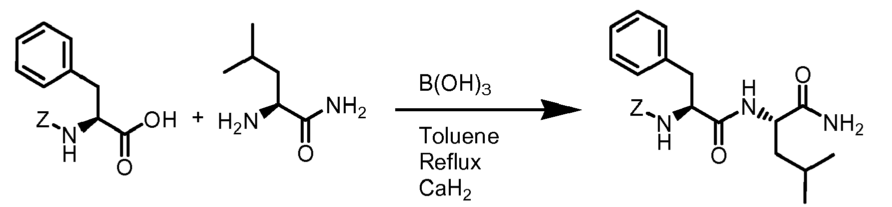

To 187 mg H-Leu-NH₂ (1 mmol) were added 299 mg Z-Phe-OH (1 mmol, 1 equiv.), 3 mg boric acid (0.05 mmol, 5 mol%) and 20 mL toluene. This mixture was heated under reflux for 16 h using a dropping funnel with pressure equalizer filled with cotton wool and 3 g of CaH₂. HPLC (method 1) indicated that 48% Z-Phe-OH had been converted and only Z-Phe-Leu-NH₂ had been formed. Analytical HPLC (method 1) indicated a d.e. of 29%.

### EXAMPLE 12: SYNTHESIS OF Z-PHE-LEU-OME USING 3,4,5-TRIFLUOROPHENYLBORONIC ACID

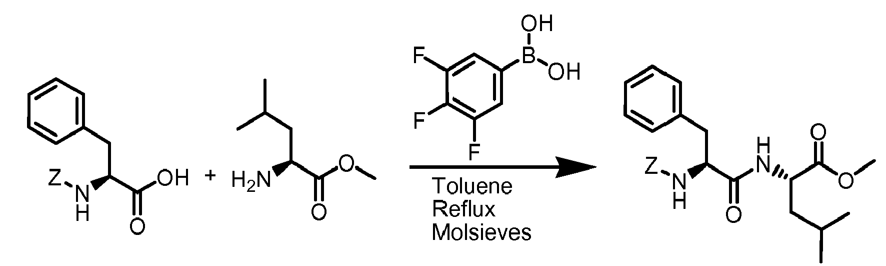

To 73 mg H-Leu-OMe (0.5 mmol) were added 150 mg Z-Phe-OH (0.5 mmol, 1 equiv.), 4 mg 3,4,5-trifluorophenylboronic acid (0.025 mmol, 5 mol%) and 10 mL toluene. This mixture was heated under reflux under reduced pressure (511 mbara) for 7 h using a Dean-Stark distilling trap filled with 10 g of 3 A molsieves. HPLC (method 1) indicated that 22% Z-Phe-OH had been converted and only Z-Phe-Leu-OMe had been formed. Analytical HPLC (method 4) indicated a d.e. of 97%.

### EXAMPLE 13: SYNTHESIS OF Z-PHE-LEU-OME USING 3,4,5-TRIFLUOROPHENYLBORONIC ACID

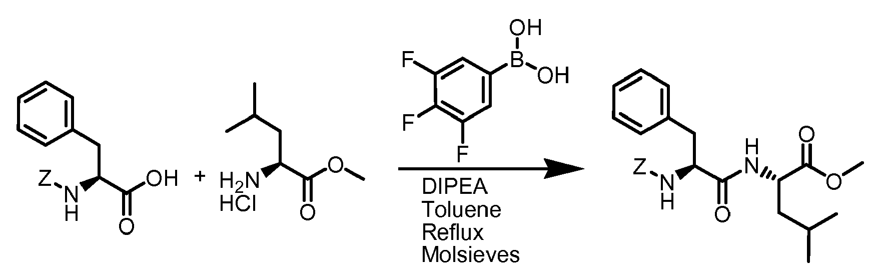

To 91 mg H-Leu-OMe·HCl (0.5 mmol) were added 150 mg Z-Phe-OH (0.5 mmol, 1 equiv.), 4 mg 3,4,5-trifluorophenylboronic acid (0.025 mmol, 5 mol%), 8.3 µL N,N-diisopropylethylamine (0,05 mmol) and 10 mL toluene. This mixture was heated under reflux under reduced pressure (511 mbara) for 24 h using a Dean-Stark distilling trap filled with 10 g of 3 A molsieves. HPLC (method 1) indicated that 16% Z-Phe-OH had been converted and only Z-Phe-Leu-OMe had been formed. Analytical HPLC (method 4) indicated a d.e. of 94%.

### EXAMPLE 14: SYNTHESIS OF Z-PHE-LEU-O^{T}BU USING 3,4,5-TRIFLUOROPHENYLBORONIC ACID

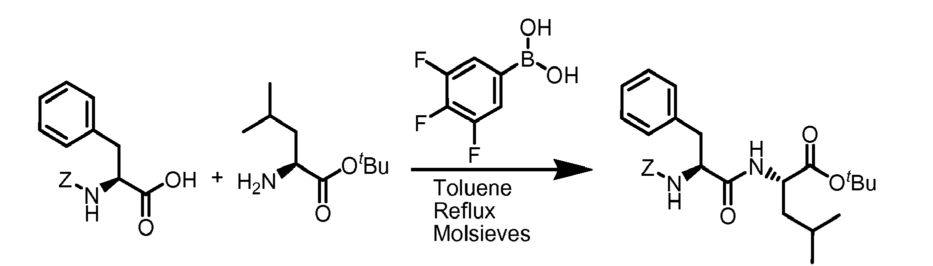

To 150 mg Z-Phe-OH (0.5 mmol) were added 468 mg H-Leu-O*^{t}*Bu (2.5 mmol, 5 equiv.), 4 mg 3,4,5-trifluorophenylboronic acid (0.025 mmol, 5 mol%), and 10 mL toluene. This mixture was refluxed for 66 h using a dropping funnel with pressure equalizer filled with cotton wool and 3 g molsieves. HPLC (method 1) indicated that 60% Z-Phe-OH had been converted and only Z-Phe-Leu-O*^{t}*Bu had been formed. Analytical HPLC (method 3) indicated a d.e. of 85%.

### EXAMPLE 15: SYNTHESIS OF Z-PHE-LEU-O^{T}BU USING 4,5,6,7-Tetrachlorobenzo[D][1,3,2]Dioxaborole

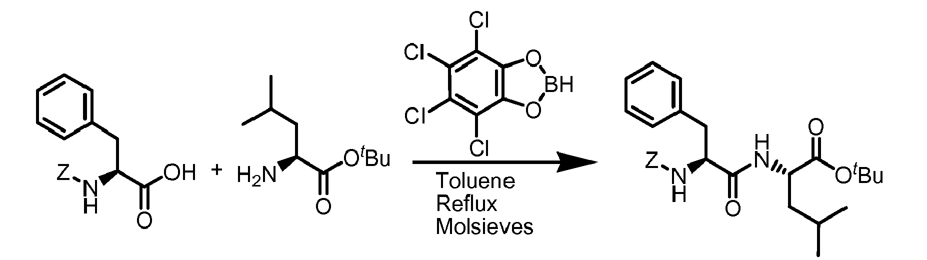

To 73 mg H-Leu-OMe (0.5 mmol) were added 150 mg Z-Phe-OH (0.5 mmol, 1 equiv.), 4 mg 4,5,6,7-tetrachlorobenzo[*d*][1,3,2]dioxaborole (0.025 mmol, 5 mol%) and 10 mL toluene. This mixture was heated under reflux under reduced pressure (511 mbara) for 17 h using a Dean-Stark distilling trap filled with 10 g of 3 A molsieves. HPLC (method 1) indicated that 40% Z-Phe-OH had been converted and only Z-Phe-Leu-OMe had been formed. Analytical HPLC (method 4) indicated a d.e. of 90%.

### EXAMPLE 16: SYNTHESIS OF Z-PHE-LEU-OME USING 3,4,5-TRIFLUOROPHENYLBORONIC ACID

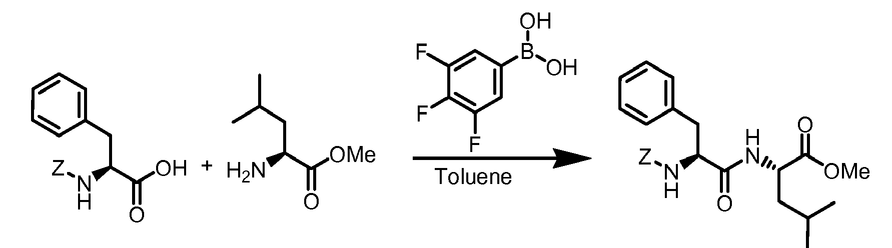

To 73 mg H-Leu-OMe (0.5 mmol) were added 150 mg Z-Phe-OH (0.5 mmol, 1 equiv.), 4 mg 3,4,5-trifluorophenylboronic acid (0.025 mmol, 5 mol%) and 10 mL toluene. The reaction was heated to distill off the wet toluene while new dry toluene was added dropwise to maintain a constant reaction volume. HPLC (method 1) indicated that after 5.5 hours 35% Z-Phe-OH had been converted and only Z-Phe-Leu-OMe had been formed. Analytical HPLC (method 4) indicated a d.e. of 84%.

## Claims

1. Method of synthesising a dipeptide, an oligopeptide or a polypeptide comprising coupling a first amino acid or peptide to a second amino acid or peptide in the presence of a boron compound comprising a boron oxygen bond, which boron compound catalyses the formation of a peptidic bond, wherein one of the stereoisomers is formed in excess of the other stereoisomer or - in case more than two stereoisomers of the peptide exist - in excess of each of the other stereoisomers, alone or taken together.

2. Method according to claim 1, wherein the first amino acid or peptide is an N-terminal protected amino acid or peptide and/or wherein the second amino acid or peptide is a C-terminal protected amino acid or peptide.

3. Method for synthesising a cyclic oligopeptide or a cyclic polypeptide, comprising forming a peptidic bond between the N-terminal amino group and the C-terminal carboxylic acid group of an oligopeptide or polypeptide, in the presence of a boron compound, which boron compound catalyses the formation of a peptidic bond, wherein one of the stereoisomers is formed in excess of the other stereoisomer or - in case more than two stereoisomers of the peptide exist - in excess of each of the other stereoisomers, alone or taken together.

4. Method according to claim 1, 2 or 3, wherein the boron compound is selected from the group of boric acid, borinic acids, boronic acids and organoboranes.

5. Method according to claim 4, wherein the boron compound comprises an aryl group to which a boron atom is directly bound.

6. Method according to claim 5, wherein the aryl group comprises at least one (further) electron withdrawing substituent, preferably at least one substituent selected from the group of halogen atoms, partially halogenated alkyl groups and fully halogenated alkyl groups.

7. Method according to claim 6, wherein the boron compound is selected from the group of 2,6-difluorophenylboronic acid, 3,4,5-trifluorophenylboronic acid, 4-bromo-2,3,5,6 tetrafluorophenylboronic acid, dimesitylene borinic acid and 4,5,6,7-tetrachlorobenzo[d][1,3,2]dioxaborole.

8. Method according to any of the preceding claims, wherein water - formed as a side-product of the coupling reaction - is removed from a reaction medium wherein the coupling is carried out.

9. Method according to any of the preceding claims, wherein the stereoisomer formed in excess of the other stereoisomer or other stereoisomers is synthesised with an enantiomeric excess or a diastereomeric excess of at least 30 %, preferably with an enantiomeric excess or a diastereomeric excess of at least 50 %, in particular with an enantiomeric excess or a diastereomeric excess of at least 65 %, more in particular with an enantiomeric excess or a diastereomeric excess of at least 85 %.

10. Method according to any of the preceding claims, wherein the boron compound is dissolved in a solution also comprising the first amino acid or peptide and the second amino acid or peptide.

11. Method according to any of the claims 1-9, wherein the boron compound is immobilised on a support.

12. Use of a boron compound comprising a boron oxygen bond as a catalyst in the preparation of a stereomerically pure peptide.

13. Use according to claim 12, wherein the boron compound is a boron compound as described in any of the claims 4, 5, 6, 7 or 11.
